# EUROPEAN PATENT APPLICATION

(11) **EP 4 644 409 A1**
(43) Date of publication of application: **05.11.2025**
(21) Application number: 23910933.3
(22) Date of filing: 29.12.2023
(51) Int. Cl.: C07K 14/43, C12N 15/80, C12N 15/29

(54) **SWEET-TASTING PROTEIN BRAZZEIN MUTANT HAVING HIGH SWEETNESS AND PREPARATION METHOD THEREFOR**

(30) Priority: 30.12.2022 CN 202211722591
(71) Applicant: Nanjing Bestzyme Bio-engineering Co. Ltd., Nanjing, Jiangsu 211100 (CN)
(72) Inventor: ZHANG, Guoxiu, Nanjing, Jiangsu 211100 (CN); LIU, Qian, Nanjing, Jiangsu 211100 (CN); ZHANG, Xudong, Nanjing, Jiangsu 211100 (CN); LI, Shaoqing, Nanjing, Jiangsu 211100 (CN); WU, Yejing, Nanjing, Jiangsu 211100 (CN)
(74) Representative: Ström & Gulliksson AB
(86) International application number: PCT/CN2023/143243
(87) International publication number: WO 2024/141019

(57) **Abstract**

The present invention provides a method for preparing Brazzein or a mutant thereof, comprising: 1) constructing an expression vector, the expression vector comprising a coding sequence of the Brazzein or the mutant thereof and a coding sequence of a leader peptide, wherein the leader peptide is derived from *Rhizopus oryzae* lipase; and 2) introducing the expression vector into a host cell. The present invention further provides Brazzein or a mutant thereof prepared by the method. The sweetness of the Brazzein or the mutant thereof is significantly higher than that of Brazzein prepared in the prior art, and the expressed protein has no obvious sweetness delay.

## Description

### Cross Reference to Related Applications

The present application claims the right of priority for Chinese patent application no. CN 202211722591.4 filed on December 30, 2022, which is incorporated herein by reference in its entirety.

### Technical Field

The present invention relates to a sweet protein and a mutant thereof, especially Brazzein and a mutant thereof. The present invention also relates to a method for preparing the Brazzein and the mutant thereof.

### Background Art

Excessive sugar intake is one of the greatest challenges to human health in the world today, and consequently, issues such as obesity, overweight and diabetes have become increasingly prominent. As health consciousness increases, low sugar and sugar reduction have become the common pursuits of both consumers and businesses. Plant sweet protein is a non-saccharide protein sweetener having high sweetness and low calories. As a sucrose substitute, the plant sweet protein does not cause diseases such as hypertension, hyperlipidaemia and diabetes, which represents great market potential in industries such as food and pharmaceuticals (Mooradian et al., The role of artificial and natural sweeteners in reducing the consumption of table sugar: A narrative review. Clin Nutr ESPEN. 2017; 18: 1-8) ^{[1]}.

Brazzein is a sweet protein extracted from fruits of *Pentadiplandra brazzeana* (Baillon), a West African climbing plant. The protein consists of 54 amino acids and is characterized as a monomeric protein with a molecular weight of 6.5 kDa (Faus, Recent developments in the characterization and biotechnological production of sweet-tasting proteins. Applied Microbiology & Biotechnology, 2000, 53(2):145-51). There are four pairs of disulfide bonds (Cys4-Cys52, Cys16-Cys37, Cys22-Cys47 and Cys26-Cys49) contained in its molecule, constituting a structural basis for good stability. Calculated by weight, the sweetness of Brazzein is 500 to 2000 times that of sucrose. Similar to sucrose, Brazzein has a clear sweet taste with a long-lasting aftertaste. Brazzein has the lowest molecular weight and the best water solubility compared to other sweet-tasting proteins. After heat treatment at 80°C for 4 hours, the aqueous solution of Brazzein remains sweet and exhibit good thermal stability and pH stability, which makes it suitable for many industrial food manufacturing processes. Therefore, Brazzein is an ideal substitute for traditional sweeteners.

However, it is difficult and expensive to obtain Brazzein by directly extracting from plants or via chemical synthesis, which fails to satisfy market demands. The efficient expression of Brazzein by means of genetic engineering is particularly significant. The *E. coli* system has been used by researchers to express Brazzein, which exists in the form of inclusion bodies after induction (Assadi-Porter et al., Efficient production of recombinant brazzein, a small, heat-stable, sweet-tasting protein of plant origin. Arch Biochem Biophys. 2000 Apr 15;376(2):252-8). Proteins require inclusion bodies to be redissolved and refolded before showing activity; however, this process is often complex, and renatured samples do not necessarily have biological activity (Lt et al., Heterologous expression and protein engineering of wheat gluten proteins. Journal of Cereal Science, 2006, 43 (3):259-274). Moreover, heterologous protein expression using inclusion bodies often requires multiple purification steps, making such systems less suitable for large-scale production. Meng et al. (Meng Shanshan et al., Expression of sweet protein Brazzein in Pichia pastoris and its application. Food and Fermentation Industries, 2020, 46(15): 6) has attempted to express Brazzein by using the *Pichia pastoris* expression system. Although the protein can be secreted and expressed, the sweetness thereof is only 40 times that of sucrose of the same quality. Obviously, the Brazzein expressed by *Pichia pastoris* has significantly insufficient activity, which is mainly due to the structural complexity of the Brazzein, especially the presence of numerous cysteine residues. Incorrect folding of a protein results in loss of protein activity during secretion. Similarly, Lee et al. (Lee et al., Expression of synthetic thaumatin genes in yeast. Biochemistry, 1988, 27(14): 5101) has also successfully expressed another sweet protein, thaumatin, by using yeast, but again yeast cells do not have the ability to process the thaumatin into a protein having a sweet tasting function.

Chinese patent CN 102498126 A discloses the expression of Brazzein by using an *Aspergillus oryzae* host, wherein the expressed Brazzein has a certain sweet tasting function, but the sweetness thereof is only about 510 times that of sucrose of the same weight; moreover, the protein has obvious sweetness delay.

### Summary of the Invention

In view of the above facts, in order to meet the growing demands for low and reduced sugar consumption, the present inventors have proposed a method for preparing Brazzein and a mutant thereof having high sweetness, wherein the sweetness is significantly higher than that of Brazzein prepared in the prior art, and the expressed protein has no obvious sweetness delay, making the protein closer to sucrose in mouthfeel. Additionally, the preparation method meets the needs of industrial production, and thanks to the significantly enhanced sweetness of the Brazzein, the production costs are significantly reduced as well.

In one aspect, provided herein is a method for preparing Brazzein or a mutant thereof, comprising:
1) constructing an expression vector, the expression vector comprising a coding sequence of the Brazzein or the mutant thereof and a coding sequence of a leader peptide, wherein the leader peptide is derived from *Rhizopus oryzae* lipase; and
2) introducing the expression vector into a host cell.

In some embodiments, the Brazzein comprises an amino acid sequence as set forth in SEQ ID NO: 1; and the mutant comprises an amino acid sequence having at least 80%, at least 85%, at least 90%, or at least 95% sequence identity to the amino acid sequence as set forth in SEQ ID NO: 1 and has a sweet taste.

In some embodiments, the Brazzein or the mutant thereof has a sweetness threshold below 100 µg/mL, preferably below 10 µg/mL, more preferably below 1 µg/mL.

In some embodiments, the mutant comprises amino acid substitutions E35D, E40A, E40D, E40K, E40R, H30R, or any combination thereof.

In some embodiments, the mutant comprises any of the following combinations of amino acid substitutions:
1) E35D/E40A;
2) E35D/E40D;
3) E35D/E40K;
4) E35D/E40R;
5) H30R/E35D/E40A;
6) H30R/E35D/E40K; and
7) H30R/E35D/E40R.

In some embodiments, the mutant comprises an amino acid sequence as set forth in any one of SEQ ID NO: 3, SEQ ID NO: 5, SEQ ID NO: 7, SEQ ID NO: 9, SEQ ID NO: 11, SEQ ID NO: 13 and SEQ ID NO: 15.

In some embodiments, the leader peptide comprises an amino acid sequence as set forth in SEQ ID NO: 17 or an amino acid sequence having at least 80%, at least 85%, at least 90%, or at least 95% sequence identity to the amino acid sequence as set forth in SEQ ID NO: 17.

In some embodiments, the Brazzein or the mutant thereof and the leader peptide are expressed in the form of a fusion protein; preferably, the leader peptide is located at the N-terminus of the fusion protein.

In some embodiments, the expression vector further comprises a coding sequence of a short peptide providing a protease cleavage site, wherein the coding sequence of the short peptide is located between the coding sequence of the leader peptide and the coding sequence of the Brazzein or the mutant thereof.

In some embodiments, the short peptide comprises amino acid sequence VALEKR.

In some embodiments, the expression vector further comprises a coding sequence of a signal peptide; preferably, the signal peptide is derived from *Aspergillus oryzae* α-amylase.

In some embodiments, the expression vector further comprises promoter and terminator sequences; preferably, the promoter and terminator are derived from an *Aspergillus oryzae* α-amylase gene.

In some embodiments, the expression vector comprises a selectable gene, preferably an *amdS* gene.

In some embodiments, the host cell is a filamentous fungus, preferably *Aspergillus oryzae,* more preferably *Aspergillus oryzae* NBRC4177.

In another aspect, provided herein is Brazzein or a mutant thereof prepared by the method described above.

In another aspect, provided herein is a nucleic acid molecule comprising a coding sequence of the Brazzein or the mutant thereof described above.

In another aspect, provided herein is an expression vector comprising a coding sequence of the Brazzein or the mutant thereof prepared by the method described above and a coding sequence of a leader peptide, wherein the leader peptide is derived from *Rhizopus oryzae* lipase.

In some embodiments, the expression vector further comprises a coding sequence of a short peptide providing a protease cleavage site, wherein the coding sequence of the short peptide is located in the 3' direction of the coding sequence of the leader peptide and in the 5' direction of the coding sequence of the Brazzein or the mutant thereof.

In some embodiments, the expression vector comprises a nucleotide sequence as set forth in any one of SEQ ID NOs: 20, 22, 24, 26, 28, 30, 32 and 34, or a nucleotide sequence having at least 80%, at least 85%, at least 90%, or at least 95% sequence identity to the nucleotide sequence as set forth in any one of SEQ ID NOs: 20, 22, 24, 26, 28, 30, 32 and 34.

In some embodiments, the expression vector further comprises a coding sequence of a signal peptide; preferably, the signal peptide is derived from *Aspergillus oryzae* α-amylase.

In some embodiments, the expression vector further comprises promoter and terminator sequences; preferably, the promoter and terminator are derived from an *Aspergillus oryzae* α-amylase gene.

In some embodiments, the expression vector described comprises a selectable gene, preferably an *amdS* gene.

In another aspect, provided herein is the use of the Brazzein or the mutant thereof described above as a food additive, a beverage additive or a medicated additive.

In another aspect, provided herein is an edible product comprising the Brazzein or the mutant thereof described above.

In some embodiments, the edible product is a food, a beverage, or a medicament.

In some embodiments, the edible product further comprises a sweetener different from the Brazzein or the mutant thereof.

In some embodiments, the sweetener includes sucrose.

In some embodiments, the beverage is a yogurt beverage or a sparkling water, such as a sugar-free sparkling water.

### Brief Description of the Drawings

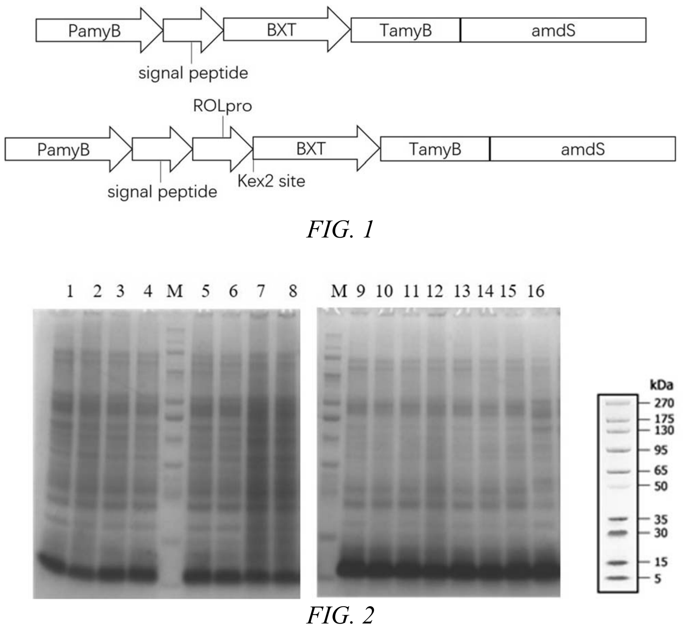

FIG. 1: A schematic map for expression elements of Brazzein and mutant thereof. In this figure, PamyB and TamyB represents the promoter and terminator of the amylase gene, respectively, "signal peptide" represents the signal peptide sequence, ROLpro represents the lipase leader peptide sequence of *Rhizopus oryzae,* Kex2 site represents the KEX2 protease cleavage site, and amdS represents a coding sequence for the acetamidase gene.
FIG. 2: SDS-PAGE detection of samples of Brazzein and mutant proteins thereof obtained from shake flask fermentation. In this figure, M represents Marker, and lanes 1 to 16 correspond to BXT, BXT-D1, BXT-D2, BXT-D3, BXT-D4, BXT-D5, BXT-D6, BXT-D7, ROLpro-BXT, ROLpro-BXT-D1, ROLpro-BXT-D2, ROLpro-BXT-D3, ROLpro-BXT-D4, ROLpro-BXT-D5, ROLpro-BXT-D6 and ROLpro-BXT-D7 proteins, respectively.

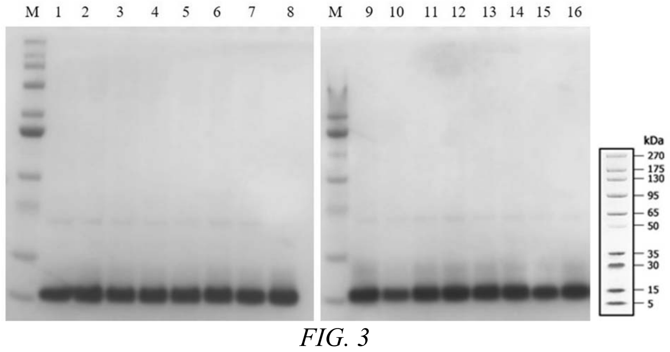

FIG. 3: SDS-PAGE detection of samples of Brazzein and mutant proteins thereof obtained from fermentation and purification. In this figure, M represents Marker, and lanes 1 to 16 correspond to BXT, BXT-D1, BXT-D2, BXT-D3, BXT-D4, BXT-D5, BXT-D6, BXT-D7, ROLpro-BXT, ROLpro-BXT-D1, ROLpro-BXT-D2, ROLpro-BXT-D3, ROLpro-BXT-D4, ROLpro-BXT-D5, ROLpro-BXT-D6 and ROLpro-BXT-D7 proteins, respectively.

### Detailed Description of Embodiments

Unless otherwise stated, all technical and scientific terms used herein have the meanings commonly understood by those of ordinary skill in the art.

Sweet protein: It refers to a protein with a strong sweetness, which is suitable for sweetening food, beverage and/or pharmaceutical products for human consumption. Calculated by weight, when compared to an 1% sucrose solution, the sweetness of the sweet protein of the present invention may be at least 1000 times, preferably at least 2000 times, more preferably at least 5000 times, and even more preferably 10000 times that of the sucrose. For the purposes of the present invention, the comparison of sweetness can be determined by human perception in testing sweetness of the protein versus a known control, wherein the testing is performed using different concentrations of proteins diluted in water or in aqueous solutions such as 20% skim milk that avoid surface adsorption.

Sweetness threshold: It is defined as the lowest concentration at which a taster identifies a sample as sweet. Sweetness potency is reported relative to sucrose.

Active ingredient: As used herein, it refers to the part of a product or composition that actually realizes what is aimed at by the product or composition, which means that the active ingredient in the sweetener is a substance that provides a sweet taste, such as a sweet protein.

Sweetener: As used herein, the term "sweetener" refers to a product or composition in a form that can be directly applicable to sweetening food, beverages and/or pharmaceutical products for human consumption. A sweetener may contain a single active ingredient, i.e. a single substance that has a sweet taste, or may contain several of such active ingredients, i.e. a blend of substances that contribute to the sweet taste. The sweetener may be an active ingredient in its substantially pure form, such as a sweet protein that is isolated from the producing cells thereof and currently exists in a form applicable to products intended for human consumption. Alternatively, the sweetener may contain other substances in addition to the active ingredient, such as, for example, a bulking agent (e.g., lactose). The sweetener may be further blended with other substances prior to application to food or beverage products, or prior to sale to ultimate consumers for household purposes such as sweetening of tea or coffee.

Brazzein: Also known as Brazzein protein, Brazzein sweet-tasting protein or Brazzein sweet protein, it refers to a sweet protein which can be extracted from fruits of *Pentadiplandra brazzeana* (Baillon) (a West African climbing plant) and described in WO 9531547, or a recombinantly produced form thereof. In nature, Brazzein occurs in three different forms, with or without a Gln residue or pyroglutamic acid at its N-terminus. In the context of the present invention, the wild-type sequence of Brazzein preferably comprises an amino acid sequence of SEQ ID NO: 1 without a Gln residue or pyroglutamic acid at its N-terminus.

Mutant: As used herein, a "mutant" of Brazzein means a sweet protein having a modified amino acid sequence compared to the amino acid sequence of Brazzein (SEQ ID NO: 1), i.e. an amino acid sequence in which one or more (several)amino acids are substituted, deleted and/or inserted.

Coding sequence: As used herein, the term "coding sequence" means a polynucleotide sequence that directly specifies the amino acid sequence of its protein product. The boundaries of the coding sequence are usually determined by an open reading frame, wherein the open reading frame usually starts with the start codon ATG or alternative start codons such as GTG and TTG, and ends with stop codons such as TAA, TAG and TGA. The coding sequences can be DNA, cDNA, RNA, or synthetic or recombinant nucleotide sequences.

Expression: Included in the context of the present invention are any steps involved in the production of the sweet protein of the present invention, including but not limited to transcription, post-transcriptional modification, translation, post-translational modification and secretion.

Expression vector: It refers to a linear or circular nucleic acid molecule construct comprising a polynucleotide encoding a protein such as the Brazzein or the mutant thereof provided herein, and the polynucleotide is operably linked to additional nucleotides provided for its expression. Such additional nucleotide sequences comprise, for example, promoters, and suitable transcription initiation and termination sequences. A "promoter" is a stretch of DNA sequence where a RNA polymerase recognizes, binds and initiates transcription, and contains a conserved sequence required for specific binding to the RNA polymerase and initiation of transcription. Most promoters are located upstream of transcription initiation sites. The promoter itself is not transcribed. In addition, the expression vector has the ability to replicate within the host, which is usually conferred by the origin of replication, and/or has a selection gene that helps to identify transformants. In general, expression vectors used in recombinant DNA technology are often in the form of "plasmids", which refer to circular double-stranded DNA loops. Obviously, vectors derived from viruses such as retroviruses and adenoviruses can also be used.

Host cell: It includes cells transformed, transfected, transduced, etc. by using nucleic acid constructs containing polynucleotides encoding sweet proteins or by expression vectors, and a sweet protein can be expressed by the cells according to the method of the present invention.

Signal peptide: The signal peptide is a short (5-30 amino acids in length) peptide chain that directs the transfer of newly synthesized proteins to a secretory pathway. It usually refers to the N-terminal amino acid sequence of a newly synthesized polypeptide chain that directs transmembrane transfer (localization) of a protein.

Leader peptide: Also known as a targeting sequence or targeting signal, it refers to a stretch of sequence that has a targeting function at the N-terminus of a protein synthesized on a free ribosome. This stretch of sequence can direct the localization of the nascent peptide to the correct cellular region, direct the protein across the membrane and finally be processed and cut off.

KEX2 protease cleavage site: Kex2 protease specifically recognizes and cleaves the dual-amino acid carboxyl-terminal peptide bond sites of Arg-Arg (arginine, Arginine, R), Lys-Arg (lysine, Lysine, K), etc.

Amino acid sequence: It is synonymous with the terms "polypeptide", "protein" and "peptide" and can be used interchangeably. A conventional one-letter or three-letter code for an amino acid residue is used, where the amino acid sequence is presented in a standard amino-to-carboxy terminal orientation (i.e., N→C).

Sequence identity: The correlation between two amino acid sequences or between two nucleotide sequences is described by the parameter "sequence identity". When aligned using the CLUSTALW algorithm with a preset parameter, a specific sequence has at least a certain percentage of amino acid residues identical to those of a specified reference sequence. The preset parameter of the CLUSTALW algorithm is deletion counts being residues that are not identical compared to the reference sequence, including deletions that occur at either terminus. For example, a variant polypeptide (with 500 amino acid residues) having five amino acid residues deleted from the C-terminus has a percent sequence identity of 99% (495/500 identical residues × 100) relative to the parent polypeptide. Such variants are encompassed by the language "variants having at least 99% sequence identity to the parent".

As used herein, the term "about" means ± 10%. As used herein, the term "about" means that the deviation above the numerical values mentioned may be at most 1%, more particularly 5%, more particularly 10%, more particularly 15%, and in some cases up to or below 20%, and the range of deviations includes integer values, and, if applicable, also includes non-integer values, forming a continuous range.

The terms "comprising", "including", "having" and combinations thereof refer to "including, but not limited to," but also refer to situations where only the listed elements are comprised.

The definitions provided herein are for ease of understanding of certain terms frequently used herein and are not meant to limit the scope of the disclosure.

Because of the structural complexity of Brazzein, in particular the presence of numerous cysteine residues, the Brazzein expressed by traditional systems such as *E. coli* and *Pichia pastoris* systems cannot fold into the correct conformation, resulting in a low sweetness of the Brazzein prepared. Ultimately, the method for preparing Brazzein by means of genetic engineering does not meet the low cost of industrial production. The present invention proposes a method for preparing Brazzein having high sweetness in response to the above-mentioned problems. In the present invention, a stretch of leader peptide sequence derived from *Rhizopus oryzae* lipase is fused with a Brazzein sequence, and the fusion sequence is expressed by using a filamentous fungal host. The resulting Brazzein and mutant proteins thereof are Brazzein having the highest sweetness ever disclosed in literature and patents to date. The highest sweetness of the Brazzein and mutants thereof prepared in the present invention is about 32.7 times that of the Brazzein prepared in the patent CN 102498126 A. By increasing the sweetness of Brazzein, the present invention makes it possible to achieve a good sweetness effect with very small additions, thereby effectively reducing the production cost of Brazzein. In addition, due to folding-promoting and secretion-promoting effects of a leader peptide from *Rhizopus oryzae* lipase, the sweet sensory properties of the Brazzein and mutants thereof prepared in the present invention are obviously improved. The Brazzein prepared in the patent CN 102498126 A has an obvious sweetness delay, wherein the sweetness can only be felt after about 1.5 seconds, which greatly limits the application performance thereof. The Brazzein and mutants thereof prepared in the present invention have no obvious sweetness delay and have an average duration of sweetness of about 25 seconds (too long duration of sweetness will cause too strong tongue-coating feeling, thus reducing people's preference). The sweet sensory properties of the Brazzein of the present invention are closer to those of sucrose, making it easier to substitute sucrose. In addition, the present invention also discloses using the prepared Brazzein in scenes such as yogurt beverages and sparkling water, which proves that the Brazzein can serve as a good substitute for sucrose and be used as a sugar substitute sweetener. The present invention provides a method for producing Brazzein from filamentous fungi, wherein the Brazzein obtained by the method is secreted extracellularly and can be easily isolated from the host strain, so that a highly pure Brazzein solution can be obtained. By applying this production method industrially, Brazzein can be produced at a much lower cost than previously disclosed production methods.

The following disclosure provides many different embodiments or examples for implementing different implementations of the present invention. In order to simplify the disclosure of the present invention, specific embodiments or examples are described below. Of course, they are merely exemplary and are not intended to limit the present invention. In addition, although the present invention provides various examples of specific processes and materials, those of ordinary skill in the art may be aware of the applicability of other processes and/or the use of other materials. Unless otherwise stated, the present invention will be practiced using conventional techniques in the fields of chemistry, molecular biology, etc. within the capabilities of those skilled in the art. In addition, unless otherwise stated, nucleic acids are written in the 5' to 3' direction from left to right herein, and amino acid sequences are written from left to right in the amino-to-carboxy direction herein.

The present invention is described below by way of illustrative specific embodiments, which do not in any way limit the scope of the present invention. In particular, reagents used in the present invention are commercially available unless otherwise specified.

### Media, reagents and experimental methods

AMDS base medium: 342.3 g/L sucrose, 20 ml/L AMDS salt solution, 10 mM acetamide, 30 mM caesium chloride, and 15 g/L agar, pH 5.5.
AMDS top medium: 342.3 g/L sucrose, 20 ml/L AMDS salt solution, 10 mM acetamide, 30 mM caesium chloride, and 10 g/L low melting point agarose, pH 5.5.
The AMDS salt solution is composed of: 26 g/L KCl, 26 g/L MgSO₄·7H₂O, 76 g/L KH₂PO₄ and 50 ml/L AMDS trace metal.
AMDS trace metal solution: 0.04 g/L Na₂B4O₇· 10H₂O, 0.4 g/L CuSO₄·5H₂O, 1.0 g/L FeSO₄·7H₂O, 1.0 g/L MnSO₄·2H₂O, 0.8 g, 1 g/L Na₂MoO₄·2H₂O and 10.0 g/L ZnSO₄·7H₂O.
TB3 low sugar medium: 3 g/L yeast extracts, 3 g/L Casamina Acids, 30 g/L sucrose and 15 g/L agar, pH 5.5.
YPG medium: 4 g/L yeast extracts, 1 g/L KH₂PO₄, 0.5 g/L MgSO₄·7H₂O and 15 g/L glucose (pH 6.0).
STC buffer is composed of: 0.8 M sorbitol, 25 mM Tris (pH 8.0) and 25 mM CaCl₂, with water making up to 1 litre.
STPC buffer is composed of: 40% PEG4000 added in the STC buffer.
Equilibration buffer (1 L): 20 mM sodium acetate buffer, pH 4.0
0.246 g of anhydrous sodium acetate was weighed, to which were added 0.97 ml of glacial acetic acid, and 800 mL of purified water. The mixture was adjusted to pH 4.0, and then volumetrically diluted to 1 L, and the prepared buffer was filtered by a 0.22 µm filter membrane.
Elution buffer (1 L): 20 mM sodium acetate buffer plus 1 M sodium chloride, pH 4.0
0.246 g of anhydrous sodium acetate was weighed, to which were added 0.97 ml of glacial acetic acid, 58.44 g of sodium chloride, and 800 mL of purified water. The mixture was adjusted to pH 4.0, and then volumetrically diluted to 1 L, and the prepared buffer was filtered by a 0.22 µm filter membrane.
Dialysis buffer (5 L): 5 mM citrate buffer, pH 4.0
2.48 g of sodium citrate and 3.18 g of citric acid were weighed, to which was added 4.8 L of purified water. The mixture was adjusted to pH 4.0 and volumetrically diluted to 5 L.

### Aspergillus oryzae genome extraction

*Aspergillus oryzae* NBRC4177 was inoculated on a TB3 low sugar medium and incubated at 30°C for 7 days until spore maturation. An appropriate amount of spore suspension (2 × 10⁷ spores/ml) was prepared and inoculated into a YPG medium liquid seed medium. The resulting mixture was incubated at 30°C and 200 rpm for 2 days until the mycelium concentration reached 4 to 5 g/L for genome extraction, with reference to the instructions of the Omega Fungal Genome Extraction Kit.

### Conventional PCR amplification:

| PCR reaction system | Volume |
|---|---|
| 5× Phusion^{™} HF Buffer | 10 µL |
| 10 mM dNTPs | 1 µL |
| Upstream primer (10 µM) | 2.5 µL |
| Downstream primer (10 µM) | 2.5 µL |
| DNA template | 1 µL |
| Phusion^{™} High-Fidelity DNA | 0.5 µL |
| Polymerase(Cat. No. F530L) | |
| ddH₂O | Making up to 50 µL |

Pre-denaturing at 98°C for 30 s; denaturing at 98°C for 10 s, annealing at 58°C for 30 s, extension at 72°C for 30 s/kb, totalling 30 cycles; final extension at 72°C for 10 min. After the reaction was complete, PCR products were identified by 1.5% agarose gel electrophoresis.

### Fusion PCR amplification

| Step 1: (No primer) | Volume |
|---|---|
| PCR reaction system | |
| 10 × PCR Buffer for KOD-Plus-Neo | 5 µL |
| 2 mM dNTPs | 5 µL |
| 25 mM MgSO₄ | 3 µL |
| Mixed DNA templates | 0.5 µL each |
| KOD-Plus-Neo(1 U/µL)(Cat. No. KOD-401) | 1 µL |
| ddH₂O | Making up to 50 µL |

Pre-denaturing at 94°C for 2 min, denaturing at 98°C for 10 s, annealing at 58°C for 30 s, extension at 68°C for 30 s/kb, totalling 10 cycles; final extension at 68°C for 10 min.

Step 2: After 10 cycles, the reaction tube was removed and 1.5 µL each of the upstream and downstream primers (10 µM) were added, followed by a PCR amplification (pre-denaturing at 94°C for 2 min, denaturing at 98°C for 30 s, annealing at 58°C for 30 s, and extension at 68°C for 30 s/kb, totalling 25 cycles; final extension at 68°C for 10 min). After the reaction was complete, PCR products were identified by 1.5% agarose gel electrophoresis.

### Aspergillus oryzae transformation

1) Slant spores of Aspergillus oryzae were inoculated into a YPG liquid medium and incubated overnight (12-16 h) at 30°C and 200 rpm to form dense and very small fungal pellets (d < 2 mm). Mycelia in the medium were filtered by sterile Microcloth, and washed 2-3 times with sterile water to finally obtain clean mycelia (about 200 mg).
2) Enzyme liquid system (5 ml) was prepared by adding 0.1 g of cellulase, 0.1 g of snail enzyme, 0.025 g of lywallzyme, and deionized water to make up to 5 ml, and performing filtration and sterilization.
3) To the mycelia prepared in step 1) was added the enzyme solution prepared in step 2), and the mixture was subjected to enzymolysis at 150 rpm and 30°C for 3 h.
4) Protoplasts were filtered by Microcloth to remove mycelial debris. The protoplasts were harvested and washed twice with STC buffer. Finally, the protoplasts were resuspended in 200-1000 microlitres of STC.
5) 5 micrograms of DNA was added to 100 microlitres of the protoplast suspension and then 200 microlitres of STPC buffer was added; and the mixture was incubated at room temperature for 20 minutes. The protoplasts were harvested and washed twice with 0.8 M sorbitol. Finally, the protoplasts were resuspended in 200 microlitres of 0.8 M sorbitol.
6) The AMDS base medium was poured in advance to form the bottom layer, and left still to solidify; the above suspension was mixed well with the AMDS top agar medium (about 50°C) and then poured to form the top layer. The ability to utilize acetamide as a sole nitrogen source was used to select transformants containing the *amdS* gene. After 5-7 days of growth at 30°C, stable transformants appeared to grow vigorously and allow colonies to form spores. The transformants were purified twice by conidia.

### Shake flask fermentation

The shake flasks containing 50 ml of YPMT media (12% of dextrin, 1% of potassium dihydrogen phosphate, 0.25% of magnesium sulphate, 2.5% of yeast extracts, 5% of peptone, 0.05% of Tween 80, the balance of water, adjusting to pH 6.0 before sterilization) were inoculated with spores of the above transformants. In order to ensure the stability of the shake flasks, the inoculation volume for all shake flasks was set as 2 ml, and the spore concentration was 2 × 10⁷/ml. The mixtures were incubated at 30°C and 200 rpm for 5 days on the same shaker.

### Aspergillus oryzae fermentation protocol

Seed culture: Spores cultured in a solid TB3 low sugar medium were transferred to shake flasks (20 g/L glycerol and 18 g/L yeast extracts) and incubated at 30°C and 250 rpm for 1 day.

Feed-batch fermentation: A 50- L tank fermentation medium (24 g/L sucrose, 10 g/L yeast extracts, 5 g/L (NH₄)₂SO₄, 2 g/L MgSO₄·7H₂O, 2 g/L K₂SO₄, 1 g/L citric acid, 2 g/L KH₂PO₄, and 0.5 ml/L trace metal solution) was adjusted to 30°C. The aeration rate was 1 vvm, and the pH was controlled at 6.0 with 10% ammonia water. The tank medium was inoculated with seed culture. At pH > 6.4, feeding (400 g/L maltose syrup and 1 g/L citric acid) was started at a rate of 3.33 g/L/h. The agitator speed was controlled to avoid low dissolved oxygen (< 20%).

### SDS-PAGE

Culture supernatants or purified samples were analysed by SDS-PAGE using a 16.5% Criterion^{™} Tris Tricine precast gel (Bio-Rad) as the SDS gel. On the gel, 20 µl of samples (10 µl of each sample mixed with 10 µl of loading buffer) were loaded, and 10 µl of Marker labelling (pre-stained SDS-PAGE standard, GenScript Biotech Co., Ltd., #M00624-250) was applied. The gel was electrophoresed in 1×SDS buffer (Bio-Rad) at a constant current of 20 mA for 80 min. The protein bands were stained with Bio-Safe Coomassie dye (Bio-Rad).

### Gene

*amdS*: This gene encodes acetamidase, an enzyme involved in the metabolism of acetamide.

### Strain

*Aspergillus oryzae* NBRC4177: available from Institute for fermentation, Osaka; 17-25 Juso Hammachi 2-Chome Yodogawa-Ku, Osaka, Japan.

### Example 1 Preparation of sequences of Brazzein and mutants thereof

Brazzein sequences from *Pentadiplandra brazzeana* (Baillon) (UniProtKB/Swiss-Prot: P56552.1) was available from the NCBI website (https://www.ncbi.nlm.nih.gov/). In the present invention, the amino acid sequence from positions 2 to 54 was selected, i.e., there was no Gln residue attached to the N-terminus. The Brazzein sequence was designated BXT, with an amino acid sequence as set forth in SEQ ID NO: 1 and a nucleotide sequence as set forth in SEQ ID NO: 2. The Brazzein variant protocol was designed targeting the Brazzein sequence by: 1) mutating glutamic acid Glu at position 35 to aspartic acid Asp, and mutating glutamic acid Glu at position 40 to alanine Ala, wherein the resulting mutant is designated BXT-D1, with an amino acid sequence as set forth in SEQ ID NO: 3 and a nucleotide sequence as set forth in SEQ ID NO: 4; 2) mutating glutamic acid Glu at position 35 to aspartic acid Asp, and mutating glutamic acid Glu at position 40 to aspartic acid Asp, wherein the resulting mutant is designated BXT-D2, with an amino acid sequence as set forth in SEQ ID NO: 5 and a nucleotide sequence as set forth in SEQ ID NO: 6; 3) mutating glutamic acid Glu at position 35 to aspartic acid Asp, and mutating glutamic acid Glu at position 40 to lysine Lys, wherein the resulting mutant is designated BXT-D3, with an amino acid sequence as set forth in SEQ ID NO: 7 and a nucleotide sequence as set forth in SEQ ID NO: 8; 4) mutating glutamic acid Glu at position 35 to aspartic acid Asp, and mutating glutamic acid Glu at position 40 to arginine Arg, wherein the resulting mutant is designated BXT-D4, with an amino acid sequence as set forth in SEQ ID NO: 9 and a nucleotide sequence as set forth in SEQ ID NO: 10; 5) mutating histidine at position 30 to arginine Arg on the basis of the mutant BXT-D1, wherein the resulting mutant is designated BXT-D5, with an amino acid sequence as set forth in SEQ ID NO: 11 and a nucleotide sequence as set forth in SEQ ID NO: 12; 6) mutating histidine at position 30 to arginine Arg on the basis of the mutant BXT-D3, wherein the resulting mutant is designated BXT-D6, with an amino acid sequence as set forth in SEQ ID NO: 13 and a nucleotide sequence as set forth in SEQ ID NO: 14; and 7) mutating histidine at position 30 to arginine Arg on the basis of the mutant BXT-D4, wherein the resulting mutant is designated BXT-D7, with an amino acid sequence as set forth in SEQ ID NO: 15 and a nucleotide sequence as set forth in SEQ ID NO: 16. The leader peptide sequence ROLpro of *Rhizopus oryzae* lipase (with the addition of a kex2 cleavage site, and having an amino acid sequence as set forth in SEQ ID NO: 18: VALEKR) and the sequences of the Brazzein or mutants thereof described above were fused, wherein the leader peptide sequence ROLpro had an amino acid sequence as set forth in SEQ ID NO: 17, and the fused sequences were designated ROLpro-BXT, ROLpro-BXT-D1 to ROLpro-BXT-D7, respectively, with amino acid and nucleotide sequences as set forth in SEQ ID NO: 19 - SEQ ID NO: 34. The fused sequences were entrusted to Nanjing GenScript Biotechnology Co., Ltd. for codon optimization and gene synthesis. The amino acid and nucleotide sequences of the leader peptide sequences of the *Rhizopus oryzae* lipase, Brazzein and mutants thereof, and fused sequences are shown in Table 1 below.

**Table 1 Amino acid and nucleotide sequences of Brazzein and mutants thereof, leader peptide sequences, and fused sequences**

| SEQ ID NO: | Sequence (5'-3') |
|---|---|
| SEQ ID NO: 1 (BXT) | DKCKKVYENYPVSKCQLANQCNYDCKLDKHARSGEC FYDEKRNLQCICDYCEY |
| SEQ ID NO: 2 (BXT) | GACAAGTGCAAGAAGGTCTACGAGAACTACCCTGTC TCCAAGTGCCAGCTCGCCAACCAGTGCAACTACGAT TGCAAGCTCGATAAGCACGCCCGCTCCGGCGAGTGC TTCTACGATGAGAAGCGCAACCTCCAGTGCATCTGC GACTACTGCGAGTAC |
| SEQ ID NO: 3 (BXT-D1) | DKCKKVYENYPVSKCQLANQCNYDCKLDKHARSGDC FYDAKRNLQCICDYCEY |
| SEQ ID NO: 4 (BXT-D1) | GACAAGTGCAAGAAGGTCTACGAGAACTACCCCGTC TCCAAGTGCCAGCTCGCCAACCAGTGCAACTACGAC TGCAAGCTCGACAAGCACGCCCGCTCCGGTGACTGC TTCTACGACGCCAAGCGCAACCTCCAGTGCATCTGCG ACTACTGCGAGTAC |
| SEQ ID NO: 5 (BXT-D2) | DKCKKVYENYPVSKCQLANQCNYDCKLDKHARSGDC FYDDKRNLQCICDYCEY |
| SEQ ID NO: 6 (BXT-D2) | GACAAGTGCAAGAAGGTCTACGAGAACTACCCCGTC TCCAAGTGCCAGCTCGCCAACCAGTGCAACTACGAC TGCAAGCTCGACAAGCACGCCCGCTCCGGTGACTGC TTCTACGACGACAAGCGCAACCTCCAGTGCATCTGC GACTACTGCGAGTAC |
| SEQ ID NO: 7 (BXT-D3) | DKCKKVYENYPVSKCQLANQCNYDCKLDKHARSGDC FYDKKRNLQCICDYCEY |
| SEQ ID NO: 8 (BXT-D3) | GACAAGTGCAAGAAGGTCTACGAGAACTACCCCGTC TCCAAGTGCCAGCTCGCCAACCAGTGCAACTACGAC TGCAAGCTCGACAAGCACGCCCGCTCCGGTGACTGC TTCTACGACAAGAAGCGCAACCTCCAGTGCATCTGC GACTACTGCGAGTAC |
| SEQ ID NO: 9 (BXT-D4) | DKCKKVYENYPVSKCQLANQCNYDCKLDKHARSGDC FYDRKRNLQCICDYCEY |
| SEQ ID NO: 10 (BXT-D4) | GACAAGTGCAAGAAGGTCTACGAGAACTACCCCGTC TCCAAGTGCCAGCTCGCCAACCAGTGCAACTACGAC TGCAAGCTCGACAAGCACGCCCGCTCCGGTGACTGC TTCTACGACCGCAAGCGCAACCTCCAGTGCATCTGCG ACTACTGCGAGTAC |
| SEQ ID NO: 11 (BXT-D5) | DKCKKVYENYPVSKCQLANQCNYDCKLDKRARSGDC FYDAKRNLQCICDYCEY |
| SEQ ID NO: 12 (BXT-D5) | GACAAGTGCAAGAAGGTCTACGAGAACTACCCCGTC TCCAAGTGCCAGCTCGCCAACCAGTGCAACTACGAC TGCAAGCTCGACAAGCGCGCCCGCTCCGGTGACTGC TTCTACGACGCCAAGCGCAACCTCCAGTGCATCTGCG ACTACTGCGAGTAC |
| SEQ ID NO: 13 (BXT-D6) | DKCKKVYENYPVSKCQLANQCNYDCKLDKRARSGDC FYDKKRNLQCICDYCEY |
| SEQ ID NO: 14 (BXT-D6) | GACAAGTGCAAGAAGGTCTACGAGAACTACCCCGTC TCCAAGTGCCAGCTCGCCAACCAGTGCAACTACGAC TGCAAGCTCGACAAGCGCGCCCGCTCCGGTGACTGC TTCTACGACAAGAAGCGCAACCTCCAGTGCATCTGC GACTACTGCGAGTAC |
| SEQ ID NO: 15 (BXT-D7) | DKCKKVYENYPVSKCQLANQCNYDCKLDKRARSGDC FYDRKRNLQCICDYCEY |
| SEQ ID NO: 16 (BXT-D7) | GACAAGTGCAAGAAGGTCTACGAGAACTACCCCGTC TCCAAGTGCCAGCTCGCCAACCAGTGCAACTACGAC TGCAAGCTCGACAAGCGCGCCCGCTCCGGTGACTGC TTCTACGACCGCAAGCGCAACCTCCAGTGCATCTGCG ACTACTGCGAGTAC |
| SEQ ID NO: 17 (ROLpro) | DDNLVGGMTLDLPSDAPPISLSSSTNSA |
| SEQ ID NO: 19 (ROLpro-BXT) | DDNLVGGMTLDLPSDAPPISLSSSTNSAVALEKRDKCK KVYENYPVSKCQLANQCNYDCKLDKHARSGECFYDE KRNLQCICDYCEY |
| SEQ ID NO: 20 (ROLpro-BXT) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC AAGAAGGTCTACGAGAACTACCCTGTCTCCAAGTGC CAGCTCGCCAACCAGTGCAACTACGATTGCAAGCTC GATAAGCACGCCCGCTCCGGCGAGTGCTTCTACGAT GAGAAGCGCAACCTCCAGTGCATCTGCGACTACTGC GAGTAC |
| SEQ ID NO: 21 (ROLpro-BXT-D1) | DDNLVGGMTLDLPSDAPPISLSSSTNSAVALEKRDKCK KVYENYPVSKCQLANQCNYDCKLDKHARSGDCFYDA KRNLQCICDYCEY |
| SEQ ID NO: 22 (ROLpro-BXT-D1) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC |
| | AAGAAGGTCTACGAGAACTACCCCGTCTCCAAGTGC CAGCTCGCCAACCAGTGCAACTACGACTGCAAGCTC GACAAGCACGCCCGCTCCGGTGACTGCTTCTACGAC GCCAAGCGCAACCTCCAGTGCATCTGCGACTACTGC GAGTAC |
| SEQ ID NO: 23 (ROLpro-BXT-D2) | DDNLVGGMTLDLPSDAPPISLSSSTNSAVALEKRDKCK KVYENYPVSKCQLANQCNYDCKLDKHARSGDCFYDD KRNLQCICDYCEY |
| SEQ ID NO: 24 (ROLpro-BXT-D2) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC AAGAAGGTCTACGAGAACTACCCCGTCTCCAAGTGC CAGCTCGCCAACCAGTGCAACTACGACTGCAAGCTC GACAAGCACGCCCGCTCCGGTGACTGCTTCTACGAC GACAAGCGCAACCTCCAGTGCATCTGCGACTACTGC GAGTAC |
| SEQ ID NO: 25 (ROLpro-BXT-D3) | DDNLVGGMTLDLPSDAPPISLSSSTNSAVALEKRDKCK KVYENYPVSKCQLANQCNYDCKLDKHARSGDCFYDK KRNLQCICDYCEY |
| SEQ ID NO: 26 (ROLpro-BXT-D3) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC AAGAAGGTCTACGAGAACTACCCCGTCTCCAAGTGC CAGCTCGCCAACCAGTGCAACTACGACTGCAAGCTC GACAAGCACGCCCGCTCCGGTGACTGCTTCTACGAC AAGAAGCGCAACCTCCAGTGCATCTGCGACTACTGC GAGTAC |
| SEQ ID NO: 27 (ROLpro-BXT-D4) | DDNLVGGMTLDLPSDAPPISLSSSTNSAVALEKRDKCK KVYENYPVSKCQLANQCNYDCKLDKHARSGDCFYDR KRNLQCICDYCEY |
| SEQ ID NO: 28 (ROLpro-BXT-D4) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC |
| | AAGAAGGTCTACGAGAACTACCCCGTCTCCAAGTGC CAGCTCGCCAACCAGTGCAACTACGACTGCAAGCTC GACAAGCACGCCCGCTCCGGTGACTGCTTCTACGAC CGCAAGCGCAACCTCCAGTGCATCTGCGACTACTGC GAGTAC |
| SEQ ID NO: 29 (ROLpro-BXT-D5) | DDNLVGGMTLDLPSDAPPISLSSSTNSAVALEKRDKCK KVYENYPVSKCQLANQCNYDCKLDKRARSGDCFYDA KRNLQCICDYCEY |
| SEQ ID NO: 30 (ROLpro-BXT-D5) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC AAGAAGGTCTACGAGAACTACCCCGTCTCCAAGTGC CAGCTCGCCAACCAGTGCAACTACGACTGCAAGCTC GACAAGCGCGCCCGCTCCGGTGACTGCTTCTACGAC GCCAAGCGCAACCTCCAGTGCATCTGCGACTACTGC GAGTAC |
| SEQ ID NO: 31 (ROLpro-BXT-D6) | DDNLVGGMTLDLPSDAPPISLSSSTNSAVALEKRDKCK KVYENYPVSKCQLANQCNYDCKLDKRARSGDCFYDK KRNLQCICDYCEY |
| SEQ ID NO: 32 (ROLpro-BXT-D6) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC AAGAAGGTCTACGAGAACTACCCCGTCTCCAAGTGC CAGCTCGCCAACCAGTGCAACTACGACTGCAAGCTC GACAAGCGCGCCCGCTCCGGTGACTGCTTCTACGAC AAGAAGCGCAACCTCCAGTGCATCTGCGACTACTGC GAGTAC |
| SEQ ID NO: 33 (ROLpro-BXT-D7) | DDNLVGGMTLDLPSDAPPISLSSSTNSAVALEKRDKCK KVYENYPVSKCQLANQCNYDCKLDKRARSGDCFYDR KRNLQCICDYCEY |
| SEQ ID NO: 34 (ROLpro-BXT-D7) | GATGATAACCTCGTCGGCGGCATGACCCTCGATCTCC CTTCCGATGCTCCTCCTATCAGCCTCTCCTCCTCCACC AACTCCGCCGTCGCTCTCGAGAAGCGCGACAAGTGC |
| | AAGAAGGTCTACGAGAACTACCCCGTCTCCAAGTGC CAGCTCGCCAACCAGTGCAACTACGACTGCAAGCTC GACAAGCGCGCCCGCTCCGGTGACTGCTTCTACGAC CGCAAGCGCAACCTCCAGTGCATCTGCGACTACTGC GAGTAC |

### Example 2 Preparation of expression elements for Brazzein and mutants thereof

### 2.1 Isolation of Aspergillus oryzae α-amylase promoter (PamyB) and signal peptide sequence thereof

According to the sequence of the amyB promoter published on GeneBank (GenBank: AP007157.1), by using the *Aspergillus oryzae* genomic DNA extracted above as a template, and using the upstream primer PasF and the downstream primer PasR1 or PasR2, a specific PCR amplification was performed to isolate the amyB promoter and the signal peptide sequence, which were designated PaS1 or PaS2 (PamyB+signal peptide). PCR products were subjected to agarose gel electrophoresis, the fragment of interest was recovered, and gel cutting and purification were carried out according to the instructions for the Axygen kit.

The sequence of the upstream primer PasF is: TAAATTTTTATATGGCGGGTGGTGG (5'-3') (the primer was synthesized by Nanjing GenScript Biotechnology Co., Ltd., the same below) (SEQ ID NO: 35).

The sequence of the downstream primer PasR1 is: GACGAGGTTATCATCAGCCAAAGCAGGTGCCGC (5'-3') (SEQ ID NO: 36).

The sequence of the downstream primer PasR2 is: CTTCTTGCACTTGTCAGCCAAAGCAGGTGCCGC (5'-3') (SEQ ID NO: 37)

### 2.2 Isolation of Aspergillus oryzae α-amylase terminator (TamyB)

According to the sequence of the amyB terminator published on GeneBank (GenBank: AP007157.1), by using the *Aspergillus oryzae* genomic DNA extracted above as a template, and using the upstream primer TayF and the downstream primer TayR, a specific PCR amplification was performed to isolate the amyB terminator sequence, which was designated TamyB. PCR products were subjected to agarose gel electrophoresis, and the fragment of interest was recovered.

The sequence of the upstream primer TayF is: TACTGCGAGTACTAGAGGGTGGAGAGTATATGATGGTA(5'-3') (SEQ ID NO: 38).

The sequence of the downstream primer TayR is: CTTCGAAGAGATACAAGTAAGTGATGGATCGCACT(5'-3') (SEQ ID NO: 39).

### 2.3 Isolation of elements of Aspergillus oryzae acetamidase coding gene (amds)

According to the *amds* gene sequence published on GeneBank (Gene ID: 5997229), by using the *Aspergillus oryzae* genomic DNA extracted above as a template, and using the upstream primer AmsF and the downstream primer AmsR, a specific PCR amplification was performed to isolate the *amds* gene sequence (comprising the promoter, gene sequence and terminator elements), which was designated amdS. PCR products were subjected to agarose gel electrophoresis, and the fragment of interest was recovered.

The sequence of the upstream primer AmsF is: GATCCATCACTTACTTGTATCTCTTCGAAGATGCCGA(5'-3') (SEQ ID NO: 40).

The sequence of the downstream primer AmsR is: GGGTGCGCCATGATAAAC(5'-3') (SEQ ID NO: 41).

### 2.4 Preparation of expression elements of Brazzein and mutants thereof by fusion of sequence elements

By using genetically synthesized sequences ROLpro-BXT, and ROLpro-BXT-D1 to ROLpro-BXT-D7 as templates, and using the upstream primer BxF1 and the downstream primer BxR, a specific PCR amplification was performed to isolate gene sequences ROLpro-BXT, and ROLpro-BXT-D1 to ROLpro-BXT-D7. By using the upstream primer BxF2 and downstream primer BxR, a specific PCR amplification was performed to isolate Brazzein BXT and mutant BXT-D1-D7 gene sequences without the lipase leader peptide sequence ROLpro. PCR products were subjected to agarose gel electrophoresis, and the fragment of interest was recovered.

The sequence of the upstream primer BxF1 is: GCACCTGCTTTGGCTGATGATAACCTCGTCGGC (5'-3') (SEQ ID NO: 42).

The sequence of the upstream primer BxF2 is: GCACCTGCTTTGGCTGACAAGTGCAAGAAGGTCTAC (5'-3') (SEQ ID NO: 43).

The sequence of the downstream primer BxR is: TATACTCTCCACCCTCTAGTACTCGCAGTAGTCGCA (5'-3') (SEQ ID NO: 44).

By using the three sequences of PaS1, ROLpro-BXT or ROLpro-BXT-D1 to ROLpro-BXT-D7, and TamyB recovered from the gel as mixed templates, and using the upstream primer PasF and the downstream primer TayR, a specific fusion PCR amplification was performed. The PCR products were subjected to agarose gel electrophoresis, and the fusion fragments PaS1-ROLpro-BXT-TamyB or PaS1-ROLpro-BXT-D1-TamyB to PaS1-ROLpro-BXT-D7-TamyB were isolated by gel recovery.

By using the three sequences of PaS2, BXT or BXT-D1 to BXT-D7, and TamyB recovered from the gel as mixed templates, and using the upstream primer PasF and the downstream primer TayR, a specific fusion PCR amplification was performed. The PCR products were subjected to agarose gel electrophoresis, and the fusion fragments PaS2-BXT-TamyB or PaS2-BXT-D1-TamyB to PaS2-D7-TamyB were isolated by gel recovery.

By using the two sequences of PaS1-ROLpro-BXT-TamyB or PaS1-ROLpro-BXT-D1-TamyB to PaS1-ROLpro-BXT-D7-TamyB, and amdS recovered from the gel as mixed templates, and using the upstream primer PasF and the downstream primer AmsR, a specific fusion PCR amplification was performed. The PCR products were subjected to agarose gel electrophoresis, and the fusion fragments PaS-ROLpro-BXT-TamyB-amdS or PaS-ROLpro-BXT-D1-TamyB-amdS to PaS-ROLpro-BXT-D7-TamyB-amdS were isolated by gel recovery to complete the construction of the expression elements of Brazzein BXT and its mutants BXT-D1-D7 with the lipase leader peptide sequence ROLpro, wherein the recovered fragments were sent to be sequenced for verification. The schematic map of the expression elements is shown in FIG. 1.

By using the two sequences of PaS2-BXT-TamyB or PaS2-BXT-D1-TamyB to PaS2-BXT-D7-TamyB, and amdS recovered from the gel as mixed templates, and using the upstream primer PasF and the downstream primer AmsR, a specific fusion PCR amplification was performed. The PCR products were subjected to agarose gel electrophoresis, and the fusion fragments PaS-BXT-TamyB-amdS or PaS-BXT-D1-TamyB-amdS to PaS-D7-TamyB-amdS were isolated by gel recovery to complete the construction of the expression elements of Brazzein BXT and its mutants BXT-D1-D7 without the lipase leader peptide sequence ROLpro, wherein the recovered fragments were sent to be sequenced for verification. The schematic map of the expression elements is shown in FIG. 1.

### Example 3 Recombinant expression of Brazzein and mutants thereof in Aspergillus oryzae

The expression element fragments of Brazzein and mutants thereof in Example 2 were transformed into *Aspergillus oryzae* strain NBRC4177, and the ability to utilize acetamide as a sole nitrogen source was used to select transformants containing the *amdS* gene. After 5 days of growth at 30°C, stable transformants appeared to grow vigorously and allow colonies to form spores. Twenty transformants were repurified twice by conidia. The shake flasks containing 50 ml of YPMT media (12% of dextrin, 1% of potassium dihydrogen phosphate, 0.25% of magnesium sulphate, 2.5% of yeast extracts, 5% of peptone, 0.05% of Tween 80, the balance of water, adjusting to pH 6.0 before sterilization) were inoculated with spores of the transformant strains, and incubated at 30°C and 200 rpm for 5 days. Culture supernatants were analysed by SDS-PAGE detection to determine whether the protein of interest was expressed and correctly cleaved, as detailed in FIG. 2. Results showed that both Brazzein and mutants thereof were expressed in Aspergillus oryzae, and Brazzein and mutants thereof with the lipase leader peptide sequence ROLpro are identical in terms of molecular weight (about 6.5 kD) to Brazzein and mutants thereof without the lipase leader peptide sequence ROLpro, indicating that the lipase leader peptide sequence ROLpro can be removed by normal cleavage. The expression level of Brazzein and mutants thereof with the lipase leader peptide sequence ROLpro is significantly higher than that of Brazzein and mutants thereof without the lipase leader peptide sequence ROLpro, indicating that the lipase leader peptide sequence ROLpro contributes to the secretory expression of Brazzein. The best transformants were selected for fermentation in a 50-litre tank, using a fermentation method as described above. The fermentation was performed at 30°C for 150 hours, and then the contents in the tank was discharged. The fermentation broth at different periods was collected and tested for protein concentration, and results showed that the protein expression in the final fermentation broth was on average around 10 g/L.

### Example 4 Purification of fermented samples of Brazzein and mutants thereof

The fermentation broth from Example 3 was filtered by a 0.22 µm filter, then the resultant was loaded onto ion exchange chromatography column SP Seplife XL (Suzhou Sunresin Biotechnology Co., Ltd.) equilibrated with 20 mM sodium acetate buffer (pH 4.0). The protein of interest was eluted with 20 mM sodium acetate buffer (pH 4.0) containing 1 M sodium chloride. The collected protein of interest was placed in a dialysis bag with a molecular weight cut-off of 3500 and dialyzed against deionized water at 4°C for 24 h, and the solution was changed three times during this process. The purified sample was dried and concentrated by lyophilization, and the resulting powder was weighed, sealed and stored in a refrigerator at 4°C. The purified protein was detected by SDS-PAGE, with the results as shown in FIG. 3. The concentration of the protein of interest was determined by the Coomassie brilliant blue method, and the sweetness activity of the protein was initially determined by tasting the dialyzed solution. This batch of purified proteins were found to be about 95% pure.

### Example 5 Determination of sweetness threshold for Brazzein and mutant proteins thereof

Since the recombinant Brazzein of the present invention is not a sugar-based compound, it is impossible to measure the sweetness by using a saccharometer. Therefore, the sweetness threshold is measured using human sensory evaluation. For most people, the sweetness threshold for sugar in soft drinks is 0.32%-1.0%. To assess the sweetness threshold for each solution, an 1% sucrose solution is used as the solution with minimum concentration at which sweetness is felt, i.e., the sweetness threshold is 10000 µg/mL. The sweetness potency of the Brazzein described herein relative to sucrose is generally in a range of 1: 500 to 1: 16,000. The samples tested included naturally extracted Brazzein (homemade, about 95% pure), recombinant Brazzein and mutants thereof, sucrose and mineral water. The protein was diluted in a gradient from 0.5 to 20 µg/ml by diluting the dry protein powder with mineral water (pH 6.9). Ten healthy evaluators (five males and five females) aged between 30 and 50 participated in the assessment, five of whom were trained wine tasters. Tasting started at the lowest concentration, with concentration increased by 1 µg/ml each time until sweetness was felt; then based on this concentration, the concentration was reduced by 0.1 µg/ml at a time until sweetness was not felt; and the sweetness threshold of the sample was determined by gradually decreasing the concentration gradient in such a way. 20 ml of samples were tested randomly compared to sugar solutions. Before each analysis, the evaluator was asked to rinse his/her mouth with water and eat the neutral crackers until there was no residual taste. The test solution was held in oral cavity for at least 10 seconds. Thereafter, evaluators provided scores against samples on the basis of their responses between 0 and 10; 0 - no sweet feeling, 10 - highly sweet. The final results for each protein were averaged for 10 individuals. The sweetness factor of Brazzein and mutants thereof relative to 1% sucrose = 10000/(sample sweetness threshold). The results are shown in Table 2.

It can be seen from results that the sweetness of Brazzein and mutants thereof prepared by adding the lipase leader peptide sequence ROLpro is significantly improved. For BXT, the addition of the lipase leader peptide sequence ROLpro achieved about 4.7-fold increase in sweetness. For BXT-D1, the addition of the lipase leader peptide sequence ROLpro achieved about 11.6-fold increase in sweetness. For BXT-D2, the addition of the lipase leader peptide sequence ROLpro achieved about 10-fold increase in sweetness. For BXT-D3, the addition of the lipase leader peptide sequence ROLpro achieved about 13.6-fold increase in sweetness. For BXT-D4, the addition of the lipase leader peptide sequence ROLpro achieved about 12-fold increase in sweetness. For BXT-D5, the addition of the lipase leader peptide sequence ROLpro achieved about 9.7-fold increase in sweetness. For BXT-D6, the addition of the lipase leader peptide sequence ROLpro achieved about 14.5-fold increase in sweetness. For BXT-D7, the addition of the lipase leader peptide sequence ROLpro achieved about 13.2-fold increase in sweetness. It can be seen from the above examples that the addition of the lipase leader peptide sequence ROLpro results in a significant increase in the expression and sweetness of Brazzein and mutant proteins thereof. The sweetness of Brazzein expressed in an Aspergillus oryzae host in patent CN102498126A is only 510 times that of sucrose of same weight, and the highest sweetness of Brazzein and mutants thereof prepared in the present invention is around 32.7 times that of the Brazzein in patent CN102498126A. The Brazzein and mutant proteins thereof prepared in the present invention are Brazzein having the highest sweetness ever disclosed in literature and patents to date. The present invention also attempted a 50-L tank scale fermentation, demonstrating that the preparation of the Brazzein and mutants thereof of the present invention is industrially feasible.

In order to further test the sweetness sensory characteristics of Brazzein and mutants thereof, the present invention diluted Brazzein and mutants thereof into an aqueous solution corresponding to the sweetness of a 6% sucrose aqueous solution in accordance with the sweetness factor and then evaluated the sweetness characteristics thereof. The evaluation was performed based on 2 indicators, sweetness delay and duration of sweetness. The results showed that, for Brazzein and mutants thereof prepared without the addition of the lipase leader peptide sequence ROLpro, the average sweetness delay was around 1.5 s and the average duration of sweetness was around 40 s. However, Brazzein and mutants thereof prepared by adding the lipase leader peptide sequence ROLpro had no obvious sweetness delay, and had an average duration of sweetness of about 25 seconds (too long duration of sweetness will cause too strong tongue-coating feeling, thus reducing people's preference), which was close to the sensory characteristics of sucrose. This indicates that the addition of the lipase leader peptide sequence ROLpro can eliminate the sweetness delay and shorten the duration of sweetness, making these proteins organoleptically closer to sucrose.

**Table 2. Determination results of sweetness threshold of Brazzein and mutants thereof**

| Protein | Concentration (µg/mL) corresponding to threshold | Sweetness fold relative to 1% sucrose |
|---|---|---|
| Naturally extracted Brazzein | 20 ± 3.9 | 501 |
| BXT | 76.9 ± 14.8 | 130.0 |
| ROLpro-BXT | 16.3 ± 2.9 | 613.5 |
| BXT-D1 | 17.4 ± 3.4 | 574.7 |
| ROLpro-BXT-D1 | 1.5 ± 0.3 | 6666.7 |
| BXT-D2 | 27.0 ± 4.9 | 370.4 |
| ROLpro-BXT-D2 | 2.7 ± 0.5 | 3703.7 |
| BXT-D3 | 21.7 ± 4.4 | 460.8 |
| ROLpro-BXT-D3 | 1.6 ± 0.3 | 6250 |
| BXT-D4 | 14.4 ± 3.1 | 694.4 |
| ROLpro-BXT-D4 | 1.2 ± 0.2 | 8333.3 |
| BXT-D5 | 11.6 ± 2.7 | 862.1 |
| ROLpro-BXT-D5 | 1.2 ± 0.2 | 8333.3 |
| BXT-D6 | 8.7 ± 1.6 | 1149.4 |
| ROLpro-BXT-D6 | 0.6 ± 0.1 | 16666.7 |
| BXT-D7 | 7.9 ± 1.5 | 1265.8 |
| ROLpro-BXT-D7 | 0.6 ± 0.1 | 16666.7 |

### Example 6 Substituting of sucrose in yogurt beverage

The taste preference of Brazzein BXT-D6 and BXT-D7 prepared by adding the lipase leader peptide sequence ROLpro in yogurt was evaluated by a sensory evaluation panel of 10 people. A yogurt product containing 4.6 g/100 g of milk fat and 4.0 g/100 g of milk protein (simplelove sugar-free - original flavour yogurt) was diluted into a yogurt product containing 3.2 g/100 g of milk protein. 4% sucrose was then added to the diluted yogurt product to form a standard sample group. It was found that in yogurt, the sweetness of 0.27 to 6.67 mg/100 g of Brazzein BXT-D6 and BXT-D7 corresponded to the sweetness obtained by adding 4% ± 1% sucrose, and the overall preference score was comparable to that of the standard sample group. Therefore, under the existing condition (80% simplelove sugar-free - original flavour yogurt), this batch of Brazzein can replace 4% sucrose, and there is no obvious decrease in the taste preference.

### Example 7 Substituting of sugar in white peach sugar-free sparkling water

The sparkling water preference was evaluated by a sensory evaluation panel of 10 people. 3.8 g of erythritol, 0.04 g of white peach essence, 0.1 g of citric acid, 2.5 to 62.5 ppm of Brazzein BXT-D6 and BXT-D7 (prepared by adding the lipase leader peptide sequence ROLpro) were accurately weighed and completely dissolved in 15 g of sugar-free soda water, and then 85 g of sugar-free soda water was added and mixed evenly to complete the preparation of the white peach sugar-free sparkling water. It was found that the white peach sugar-free sparkling water to which Brazzein was added exhibited a sweetness preference score equivalent to that of the white peach sugar-free sparkling water available on the market. Therefore, under the condition of the white peach sugar-free sparkling water, Brazzein can be used as a sugar substitute sweetener.

In the description of the present specification, the description of references to terms such as "an embodiment", "some embodiments", "examples", "specific examples", or "some examples" means that specific features, structures, materials or characteristics described in combination with the embodiment(s) or example(s) are comprised in at least one embodiment or example of the present invention. In the present description, the schematic expressions of the above terms should not be construed as necessarily referring to the same embodiment or example. Furthermore, the specific features, structures, materials or characteristics described may be combined in a suitable manner in any one or more embodiments or examples. In addition, those skilled in the art may integrate and combine different embodiments or examples described in the present description.

Although the embodiments of the present invention have been shown and described above, it should be understood that the above-mentioned embodiments are merely exemplary and should not be construed as limiting the present invention. Those of ordinary skill in the art would have made changes, modifications, replacements and variations to the above-mentioned embodiments within the scope of the present invention.

## Claims

1. A method for preparing Brazzein or a mutant thereof, **characterized by** comprising:
1) constructing an expression vector, the expression vector comprising a coding sequence of the Brazzein or the mutant thereof and a coding sequence of a leader peptide, wherein the leader peptide is derived from *Rhizopus oryzae* lipase; and
2) introducing the expression vector into a host cell.

2. The method of claim 1, **characterized in that** the Brazzein comprises an amino acid sequence as set forth in SEQ ID NO: 1; and the mutant comprises an amino acid sequence having at least 80%, at least 85%, at least 90%, or at least 95% sequence identity to the amino acid sequence as set forth in SEQ ID NO: 1 and has a sweet taste.

3. The method of claim 1 or 2, **characterized in that** the Brazzein or the mutant thereof has a sweetness threshold below 100 µg/mL, preferably below 10 µg/mL, more preferably below 1 µg/mL.

4. The method of any one of claims 1-3, **characterized in that** the mutant comprises amino acid substitutions E35D, E40A, E40D, E40K, E40R, H30R, or any combination thereof.

5. The method of any one of claims 1-4, **characterized in that** the mutant comprises any of the following combinations of amino acid substitutions:
1) E35D/E40A;
2) E35D/E40D;
3) E35D/E40K;
4) E35D/E40R;
5) H30R/E35D/E40A;
6) H30R/E35D/E40K; and
7) H30R/E35D/E40R.

6. The method of any one of claims 1-5, **characterized in that** the mutant comprises an amino acid sequence as set forth in any one of SEQ ID NO: 3, SEQ ID NO: 5, SEQ ID NO: 7, SEQ ID NO: 9, SEQ ID NO: 11, SEQ ID NO: 13 and SEQ ID NO: 15.

7. The method of any one of claims 1-6, **characterized in that** the leader peptide comprises an amino acid sequence as set forth in SEQ ID NO: 17 or an amino acid sequence having at least 80%, at least 85%, at least 90%, or at least 95% sequence identity to the amino acid sequence as set forth in SEQ ID NO: 17.

8. The method of any one of claims 1-7, **characterized in that** the Brazzein or the mutant thereof and the leader peptide are expressed in the form of a fusion protein; preferably, the leader peptide is located at the N-terminus of the fusion protein.

9. The method of any one of claims 1-8, **characterized in that** the expression vector further comprises a coding sequence of a short peptide providing a protease cleavage site, wherein the coding sequence of the short peptide is located between the coding sequence of the leader peptide and the coding sequence of the Brazzein or the mutant thereof.

10. The method of any one of claims 1-9, **characterized in that** the short peptide comprises amino acid sequence VALEKR.

11. The method of any one of claims 1-10, **characterized in that** the expression vector further comprises a coding sequence of a signal peptide; preferably, the signal peptide is derived from *Aspergillus oryzae* α-amylase.

12. The method of any one of claims 1-11, **characterized in that** the expression vector further comprises promoter and terminator sequences; preferably, the promoter and terminator are derived from an *Aspergillus oryzae* α-amylase gene.

13. The method of any one of claims 1-12, **characterized in that** the expression vector comprises a selectable gene, preferably an *amdS* gene.

14. The method of any one of claims 1-13, **characterized in that** the host cell is a filamentous fungus, preferably *Aspergillus oryzae,* more preferably *Aspergillus oryzae* NBRC4177.

15. Brazzein or a mutant thereof prepared by the method of any one of claims 1-14.

16. A nucleic acid molecule, **characterized by** comprising a coding sequence of the Brazzein or the mutant thereof of claim 15.

17. An expression vector, **characterized by** comprising a coding sequence of the Brazzein or the mutant thereof prepared by the method of any one of claims 1-14 and a coding sequence of a leader peptide, wherein the leader peptide is derived from *Rhizopus oryzae* lipase.

18. The expression vector of claim 17, **characterized by** further comprising a coding sequence of a short peptide providing a protease cleavage site, wherein the coding sequence of the short peptide is located in the 3' direction of the coding sequence of the leader peptide and in the 5' direction of the coding sequence of the Brazzein or the mutant thereof.

19. The expression vector of claim 17 or 18, **characterized by** comprising a nucleotide sequence as set forth in any one of SEQ ID NOs: 20, 22, 24, 26, 28, 30, 32 and 34, or a nucleotide sequence having at least 80%, at least 85%, at least 90%, or at least 95% sequence identity to the nucleotide sequence as set forth in any one of SEQ ID NOs: 20, 22, 24, 26, 28, 30, 32 and 34.

20. The expression vector of any one of claims 17-19, **characterized by** further comprising a coding sequence of a signal peptide; preferably, the signal peptide is derived from *Aspergillus oryzae* α-amylase.

21. The expression vector of any one of claims 17-20, **characterized in that** the expression vector further comprises promoter and terminator sequences; preferably, the promoter and terminator are derived from an *Aspergillus oryzae α-*amylase gene.

22. The expression vector of any one of claims 17-21, **characterized by** further comprising a selectable gene, preferably an *amdS* gene.

23. Use of the Brazzein or the mutant thereof of claim 15 as a food additive, a beverage additive or a medicated additive.

24. An edible product, **characterized by** comprising the Brazzein or the mutant thereof of claim 15.

25. The edible product of claim 24, **characterized in that** the edible product is a food, a beverage or a medicament.

26. The edible product of claim 24 or 25, **characterized by** further comprising a sweetener different from the Brazzein or the mutant thereof.

27. The edible product of any one of claims 24-26, **characterized in that** the sweetener includes sucrose.

28. The edible product of any one of claims 24-27, **characterized in that** the beverage is a yogurt beverage or a sparkling water, such as a sugar-free sparkling water.
